# EUROPEAN PATENT APPLICATION

(11) **EP 4 631 477 A1**
(43) Date of publication of application: **15.10.2025**
(21) Application number: 25168243.1
(22) Date of filing: 03.04.2025
(51) Int. Cl.: A61F 2/36, A61F 2/38

(54) **ASSEMBLY AND METHOD FOR MAKING AN INTRAMEDULLARY SPACER**

(30) Priority: 11.04.2024 IT 202400008128
(71) Applicant: G21 S.r.l., 41039 San Possidonio (MO) (IT)
(72) Inventor: FORONI, Filippo, 41037 Mirandola (MODENA) (IT)
(74) Representative: Gagliardelli, Fabrizio

(57) **Abstract**

Described is an assembly (1) for making an intramedullary spacer (20) made of medical cement, designed to be inserted in a medullary canal, comprising a stem module (2) and a reinforcing core (4),
wherein the stem module (2) comprises an end wall (23) delimiting below a stem moulding chamber (SMC); and
wherein the reinforcing core (4) can be at least partly positioned in the stem moulding chamber (SMC).

## Description

This invention relates to the field of orthopaedic surgery.

The subject of this invention is, in particular, an assembly for making an intramedullary spacer in an operating room which can be used in orthopaedic surgery for the treatment of bone fractures or in orthopaedic surgery for revision of an articular prosthesis, following the failure of said prosthesis due to the occurrence of an infection at the implant site; further objects of this invention are a method for making an intramedullary spacer starting from said assembly and the intramedullary spacer made following the method.

In the field of orthopaedic surgery applied to the treatment of fractures, in particular broken fractures of long bones, in which several bone segments are misaligned following a traumatic event, there are prior art devices called intramedullary nails, made of metal or metal alloys, which are inserted in use inside the medullary canal of the fractured long bone. The term "long bone" means in anatomy a bone which has a main longitudinal extension relative to its other two dimensions: a long bone identifies an elongate central portion, called the diaphysis, whose length is crossed by a medullary canal in which the bone marrow is situated, delimited by two portions called the epiphyses, having globular shapes configured to articulate with other bones of the skeleton, to allow the physiological mobility of said long bone; examples of long bone are the femur, tibia, fibular and humerus.

The intramedullary nail allows the bone fragments to be correctly repositioned relative to each other, restoring the original structure of the long bone and of the respective medullary canal, allowing the stabilisation of the long bone, recalcification of the bone at the interface between bone segments and, consequently, healing of the long bone.

During the procedure, an operator positions the intramedullary nail at the medullary canal of the fractured long bone and correctly positions the bone segments relative to each other, realigning them correctly and using the intramedullary nail as a central guide. If necessary, the bone segments are stabilised and fastened to each other with the aid of metal plates and screws, thus restoring the long bone structure and the integrity of the medullary canal.

Around the intramedullary nail, the long bone re-calcifies correctly and recovers its original structural integrity.

However, sometimes infections may occur at the implant site.

If the infection is treatable with antibiotics, the treatment protocol is often long and debilitating, slowing down and compromising the process of healing of the bone and restoring the original medullary canal due to the strong inflammatory state that is created in the implant site following the infection.

On the other hand, in the event of the proliferation of resistant antibiotic bacteria or when the proliferation of bacteria is particularly serious, the administration of antibiotics is not sufficient and forces the operator to intervene, under far from optimum intervention conditions, to remove the intramedullary nail and to manually clean the area, if necessary removing part of bone and marrow inevitably compromised by the proliferation of the infection.

In both cases, the prior art has obvious limitations and the approaches described risk lengthening and adversely affecting the process of recalcification of the fractured bone, and it can therefore be much improved, in order to guarantee a faster and more effective healing of the fractured bone.

Considering the above-mentioned field of orthopaedic revision surgery, the term spacer means a device made of medical cement, that is to say, bone cement impregnated with antibiotics, designed for implantation at a bone and, in detail, inserted at least partly in a medullary canal, in place of an infected articular prosthesis, in order to temporarily restore the function of the implant seat and to treat the active infection in said implant seat.

The spacers are in fact used during surgical protocols, known as "revisions", in two steps, wherein a first step comprises the explanation of the infected prosthesis and its replacement with the corresponding spacer, and wherein a second step, when the infection is completely treated, comprises the removal of the spacer and the insertion of a new joint prosthesis.

With particular reference to the knee joint, the spacers comprise a femoral component, called femoral spacer, and a tibial component, called tibial spacer, which are configured to articulate so as to restore, as much as possible, the physiological functionality of the knee joint, at the femoral condyles and the tibial plateau, respectively.

The tibial spacer and the femoral spacer are obtained by moulding medical cement in an operating room inside, respectively, a femoral module and a tibial module, an operation which is performed by an orthopaedic surgeon at the same time as the explanting of the infected prosthesis.

Considering in particular the femoral spacers, there are currently prior art moulds defining a moulding chamber having a C-shaped transversal cross-section, shaped for making a condylar component corresponding to the distal femoral condyles. The prior art solutions, in detail, have a first half-mould and a second half-mould, which can be coupled to the first half-mould, to identify said moulding chamber.

During the operation, the surgeon injects a quantity of liquid medical cement in the main chamber: once the solidification of the medical cement inside the moulding chamber is completed, the mould is broken or removed to allow the extraction of the femoral spacer, which, therefore, has a C-shaped section and a shape corresponding to the distal femoral condyles. The spacer obtained is positioned at the distal end of the femur and constrained to said end interposing a further quantity of liquid medical cement.

However, the prior art solutions have obvious limitations.

Firstly, the femoral spacer made by means of the prior art moulds and cemented to the distal portion of the femur can be considerably improved in terms of the stability of connection to the bone of the patient's femur.

In order to favour the stabilisation, during the surgery the surgeon can shape by hand a stem starting from a quantity of fluid medical cement: once solidified, the stem shaped by hand is then constrained to the moulded condylar component, interposing a further quantity of fluid bone cement. The femoral spacer obtained is positioned at the distal epiphysis of the femur, inserting the shaped stem into the femoral canal.

Although this latter solution is more stably positioned with respect to the femoral bone, it is not very adaptable to the specific anatomy of the patient and, in particular, to the shape of bone and tissues at the distal portion of the femur; moreover, the outcome is approximate and dependent strictly on the manual skills of the individual surgeon and of the individual implant procedure.

The technical purpose of the invention is therefore to provide an assembly, a kit and a method for making an intramedullary spacer in an operating room which are able to overcome the obvious limitations of the prior art. The technical purpose of the invention is also to provide an intramedullary spacer made in an operating room using said assembly and kit and following said method.

The technical purpose and the aims specified are achieved by an assembly and a kit for making an intramedullary spacer in an operating room, a relative method and the intramedullary spacer made by means of said assembly and said kit and by said method, comprising the technical features described in one or more of the appended claims.

Further features and advantages of the invention are more apparent in the non-limiting description which follows of a preferred non-limiting embodiment of an assembly and a kit for making an intramedullary spacer in an operating room, a relative method for making it and the intramedullary spacer made by means of said assembly and by means of said method.

The description is set out below with reference to the accompanying drawings which are provided solely for purposes of illustration without restricting the scope of the invention and in which:
- Figure 1 shows an exploded view of an assembly for making an intramedullary spacer according to this invention;
- Figure 2 shows an assembled isometric view of the assembly of Figure 1;
- Figure 3A shows a side view of a stem module included in the assembly of Figure 1;
- Figure 3B shows a side view of a half-module for the stem included in the assembly of Figure 1;
- Figure 4A shows an isometric view of a closing element included in the assembly of Figure 1;
- Figure 4B shows a side view of the closing element of Figure 4A;
- Figure 5 shows a reinforcing element included in the assembly of Figure 1;

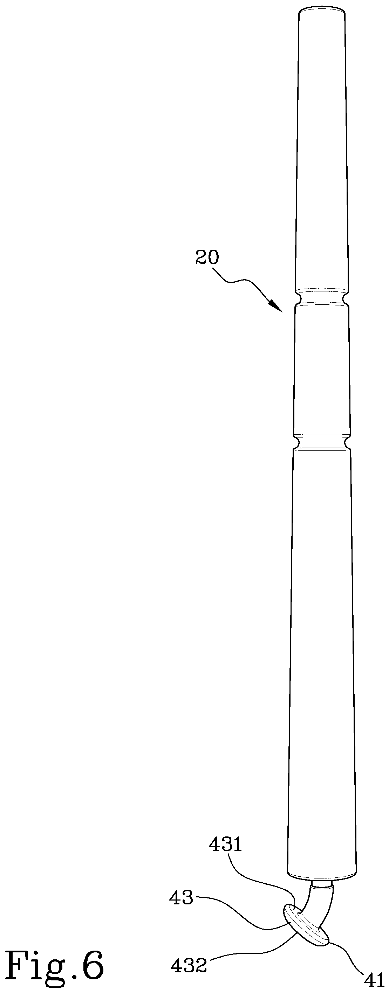

- Figure 6 shows a femoral stem made by means of the assembly of Figure 1;
- Figure 7 shows a condylar component;
- Figure 8A shows an isometric view of a coupling between the reinforcing element of Figure 5 and the condylar component of Figure 7;
- Figure 8B shows a side view of the coupling of Figure 8A.

The first object of this invention is an assembly 1 for making an intramedullary spacer 20 in an operating room comprising a metal reinforcing core 4.

Preferably, but not exclusively, said intramedullary spacer 20 is made of medical cement, with the addition of an active compound, such as an antibiotic drug for the treatment of infections which have arisen at the implant site.

The intramedullary spacer 20 according to this invention is in use designed to be inserted in a medullary canal of a long bone, to prevent the occurrence of infections or to treat infections which have arisen in bone regions surrounding the medullary canal or in tissues adjacent to the bone in which said intramedullary spacer is inserted.

The assembly 1 according to this invention comprises a stem module 2 and a reinforcing core 4. The stem module 2 comprises an end wall 23 delimiting below a stem moulding chamber SMC for making an intramedullary spacer 20 which, in use, can be inserted inside a medullary canal. The end wall 23, in other words, is located at an end of the stem module 2, to delimit below the stem moulding chamber SMC.

The assembly 1 according to this invention also comprises a reinforcing core 4 which can be at least partly positioned in the stem moulding chamber SMC, to make an intramedullary spacer 20 reinforced by said reinforcing core 4.

The reinforcing core 4 is preferably made of metal material or metal alloy. The stem moulding chamber SMC, as described below, is designed to receive a quantity of fluid medical cement and to allow the solidification to obtain the intramedullary spacer 20, suitable for being inserted in a medullary canal of a patient. The stem moulding chamber SMC is shaped internally to reproduce during moulding the anatomy of a specific medullary canal.

Inside the human body, the medullary canals have a spongy structure and typically have an elongate extension between two end portions. In fact, considering a long bone, it has an elongate part, called the diaphysis, delimited by two articulation ends, called the epiphyses. The medullary canal therefore extends inside the diaphysis and is delimited above and below by the epiphyses.

Moreover, the long bone has cortical bone tissue externally, that is to say, hard and compact, whilst, internally, the medullary canal is spongy and resistant to a considerably lesser extent.

For this reason, the intramedullary spacer 20 is shaped to be inserted during implantation in the less resistant portion of the long bone, that is to say, inside the medullary canal, without touching the outer cortical bone.

In order to allow the intramedullary spacer 20 to be easily inserted into the medullary canal, the intramedullary spacer 20 must have a similarly elongate extension relative to the canal. The stem module 2 therefore has internally an elongate moulding surface, elongate between two ends. One of the ends is formed by the end wall 23.

Therefore, the end wall 23 is intended in use to make an end portion of the intramedullary spacer 20.

According to a first embodiment of the assembly 1 according to this invention, the reinforcing core 4 is completely inserted inside the stem moulding chamber SMC, to make a reinforced intramedullary spacer 20, which in use can be inserted in a medullary canal.

The intramedullary spacer 20 obtained by means of said assembly 1 according to its first embodiment is a temporary intramedullary nail 20 to treat a broken fracture and to prevent or cure infections at the intramedullary canal or of the tissues surrounding the bone whose integrity is to be restored.

In other words, the temporary intramedullary nail 20 thus obtained can be advantageously used to align two or more bone fragments resulting from a broken fracture of a long bone, restoring the original structure of the long bone and of the respective medullary canal, allowing the recalcification and treating any infections which arise at the implant site.

The temporary intramedullary nail 20 is inserted by an operator during surgery in the medullary canal of the fractured bone and the bone segments resulting from the broken fracture are aligned following the longitudinal extension of said temporary intramedullary nail 20, or, in other words, they are positioned in such a way that the temporary intramedullary nail 20 is correctly inserted inside the medullary canal of the bone.

Once the infection has been eliminated in the area of interest and the integrity of the bone to be treated has been at least partially restored, the operator can remove the temporary intramedullary nail 20, pulling it out of the medullary canal of interest, to replace it with a prior art intramedullary metal nail, which will be kept in the implant site.

According to a second embodiment, the assembly 1 according to this invention has an end wall 23, having an opening 231 for putting in communication the stem moulding chamber SMC with an outside environment. The opening 231 is, in other words, made in the end wall 23 and is therefore located at an end of the stem module 2.

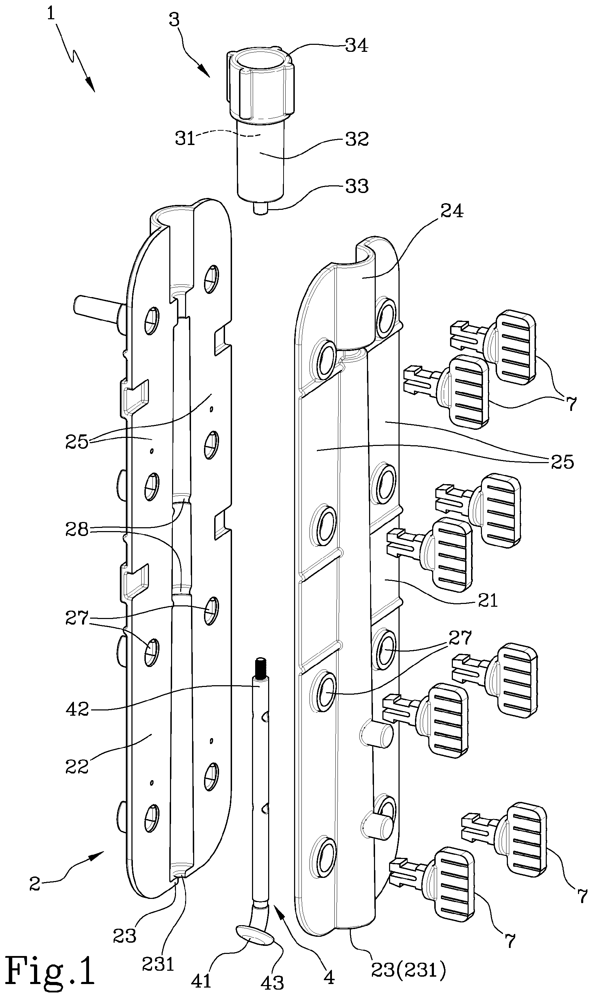

According to the second embodiment, the reinforcing core 4 is partly located inside the stem moulding chamber SMC and partly protruding from said stem moulding chamber SMC through the opening 231, to make an intramedullary spacer 20 having the reinforcing core 4 protruding (Figure 1).

With particular, but not exclusive, reference to the bone of the femur, the assembly 1 according to the second embodiment of this invention makes it possible to obtain a femoral stem 20 having a reinforcing core 4 protruding from said femoral stem 20, which in use can be inserted in the femoral medullary canal.

Advantageously, the femoral stem 20 can be used to restore the integrity of the femoral bone in the event of a broken femur fracture. Alternatively, or in addition, the femoral stem 20 obtained through the assembly 1 according to the second embodiment can be used to be constrained to a condylar component 30 through a quantity of bone cement to form a femoral spacer 10 of a temporary knee prosthesis.

In other words, the intramedullary spacer 20 having the protruding reinforcing core 4 can be positioned in the distal femoral canal and can be used as a femoral stem 20 for positioning and stabilising a femoral spacer 10 comprising the above-mentioned condylar component 30 connected to the femoral stem 20.

The femoral spacer 10 thus obtained allows the functionality of the knee joint to be temporarily restored, articulating it with a respective tibial spacer positioned at the tibia.

This invention does not exclude intramedullary spacers which can be used as positioning and stabilising stems for temporary spacers of joints different from the knee joint.

For example, but not exclusively, the assembly, the kit and the method according to the invention can be used in the production of a tibial spacer of a temporary knee prosthesis or of a humerus spacer of a temporary shoulder prosthesis.

In order to simplify the description, when describing the features of the assembly 1 according to this invention, particular but not exclusive reference will be made to the assembly 1 for making a femoral stem 20. However, the features described are intuitively also applicable to an assembly 1 for making a temporary intramedullary nail 20 in a medullary canal of a bone different from the femur.

For this reason, the assembly 1 according to this invention will be described in detail below with particular reference to the bone of the femur and to an intramedullary spacer 20 which can be inserted in the femoral medullary canal. The term "proximal" will be used in the invention to indicate elements or parts of elements which, in use, are closest to the hip joint or devices which make said elements or parts of elements closest to the hip joint, whilst the term "distal" will be used to indicate elements or parts of elements which, in use, are furthest from the hip joint or devices which make the elements or parts of elements furthest from the hip joint. With reference to the above, the assembly 1 according to this invention comprises the stem module 2 delimiting the stem moulding chamber SMC, set up to receive the bone cement in a fluid form, said stem chamber having the end wall 23 delimiting below the stem moulding chamber SMC and having, according to an embodiment, the opening 231. Moreover, the stem module 2 comprises the metallic reinforcing core 4 which can be positioned inside or partly inside the stem moulding chamber SMC to obtain alternative embodiments of the assembly 1 and of the intramedullary spacer 20.

The assembly 1 according to the invention further comprises a closing element 3 which can be coupled to the stem module 2 to delimit above the stem moulding chamber SMC.

In other words, the stem module 2 defines inside it the stem moulding chamber SMC and the closing element 3 can be coupled to said stem module 2 to delimit the stem moulding chamber SMC. The stem moulding chamber SMC is therefore defined by the shape of an inner surface of the stem module 2.

More in detail, the stem moulding chamber SMC is elongate, that is to say, it has a main extension along a specific longitudinal direction. More in detail, the stem moulding chamber SMC defines an inner moulding surface which is elongate in shape, that is to say, having an elongate extension between the end wall 23 and the closing element 3.

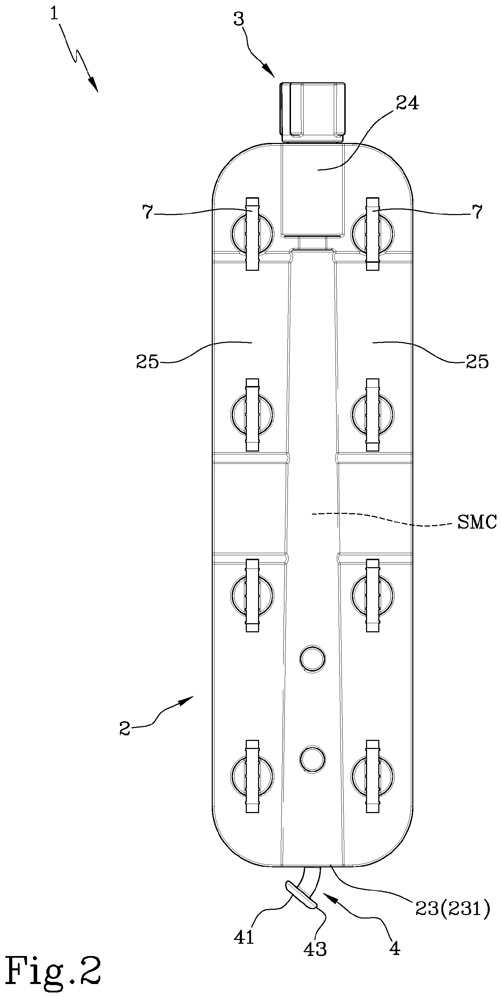

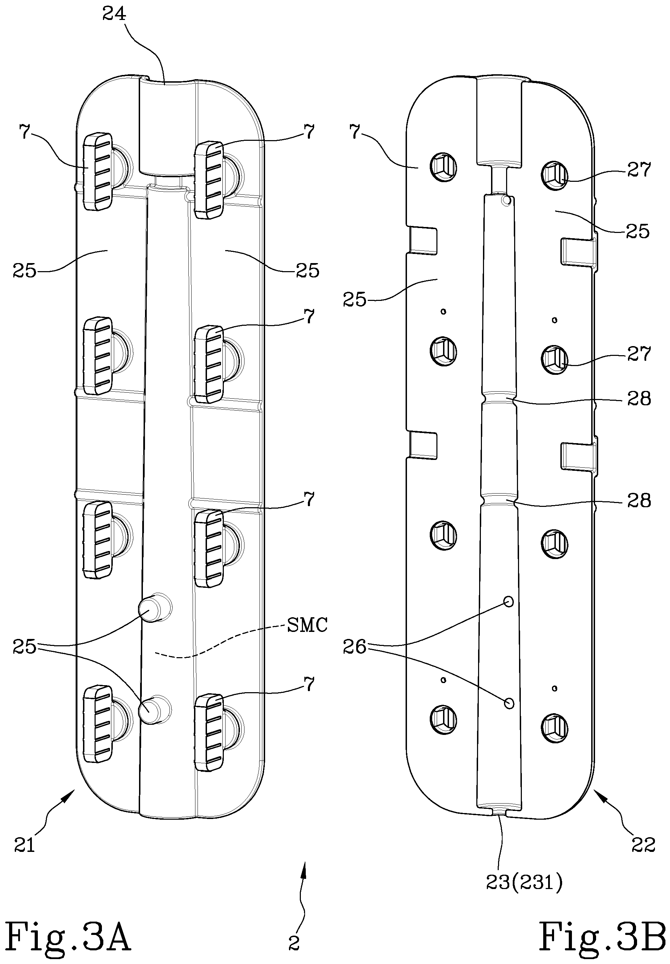

According to a first embodiment, illustrated in Figures 2 and 3A, the femur moulding chamber SMC has a substantially cylindrical shape and may have a constant or variable diameter. The stem moulding chamber SMC shaped in this way allows a femoral spacer 10 to be obtained having a femoral stem 20 with an elongate shape, and preferably substantially cylindrical, with a constant or variable diameter.

According to a second embodiment, not shown in the accompanying drawings, the stem moulding chamber SMC has a first stretch, or proximal stretch, and a second stretch, or distal stretch, which are parallel to the longitudinal direction and offset from each other, said proximal stretch and said distal stretch being preferably connected by an intermediate stretch. Preferably, said proximal stretch and said distal stretch of the femur moulding chamber SMC are cylindrical and connected by the intermediate stretch: the intermediate stretch corresponds to a substantially cylindrical connection between the proximal stretch and the distal stretch. Preferably, the proximal stretch, the distal stretch and the intermediate stretch have a substantially constant transversal cross-section.

The femur moulding chamber SMC according to the second embodiment allows a spacer 10 to be obtained comprising a femoral stem 20 having overall extension in the longitudinal direction and having a proximal portion and a distal portion elongate along respective axes, said axes being substantially parallel to the longitudinal direction; in other words, said proximal portion and said distal portion are offset and connected by an intermediate connecting portion, to define an offset" between the two proximal and distal portions. Advantageously, the presence of the 'offset' allows a femoral stem 20 to be made which can be introduced and positioned in femoral canals that are not perfectly rectilinear without the risk of damaging the implant or the cortical femoral bone of the patient. In other words, the femoral stem 20 shaped in this way facilitates the positioning of a femoral spacer 10 using femoral canals which are not perfectly rectilinear, taking into account the anatomical variabilities of the patients.

According to the preferred embodiment, the assembly 1 according to this invention has a stem module 2 comprising a first stem half-module 21 and a second stem half-module 22, which can be coupled to the first stem half-module 21 to identify the femoral moulding chamber SMC. Moreover, the first and second stem half-modules 21, 22 can be coupled to define the opening 231.

Preferably, the first stem half-module 21 and the second stem half-module 22 can be coupled by means of respective flanges 25: in other words, the first and the second stem half-modules 21, 22 comprise respective flanges 25 prepared to allow the mutual moving towards each other of the first and second stem half-modules 21, 22 and to reach the desired mutual position and orientation between the first and second stem half-modules 21, 22, so as to correctly identify the stem moulding chamber SMC.

The flanges 25 are positioned on the perimeter of the respective half-module 21, 22, whilst the inner surfaces which define the stem chamber SMC are made centrally to the respective half-module 21, 22 so that, following the contact of the flanges 25, the stem moulding chamber SMC is defined inside the closed stem module 2.

Moreover, the first and second stem half-modules 21, 22 may have one or more holes 27 at the flanges 25, said holes 27 being distributed in such a way as to be next to and coaxial with the correct coupling between the first and second stem half-modules 21, 22. Said holes 27 are engaged in use by fixing means 7 to keep coupled the first and the second stem half-modules 21, 22 during the injection of the fluid medical cement and its solidification.

Further, the stem module 2 may have one or more weaknesses 28; considering a thickness which is substantially constant in the stem module 2 and, in particular, at the femur moulding chamber SMC, the weaknesses 28 correspond to portions of the stem module 2 identifying localised thinning of the above-mentioned thickness; in particular, the weaknesses 28 define thinned zones at the stem moulding chamber SMC, designed to indicate and define preferential sections of failure of the stem module 2. The weaknesses 28 preferably define a plane of failure of the stem module 2 transversal to the longitudinal direction. Preferably, the weaknesses 28 each describe structures annular to the stem moulding chamber SMC, facilitating the breakage of the stem module for 2 at a specific weakness 28.

In fact, the stem module 2, after the injection of the fluid medical cement inside the stem moulding chamber SMC, may be broken at a weakness 28, so as to obtain a femoral spacer 10 having the femoral stem 20 of the desired length: the broken portion furthest from the end wall 23 is then removed from the stem module 2.

Advantageously, the presence of a plurality of weaknesses 28 allows the assembly 1 to be used and, in particular, the stem module 2 to obtain femoral spacers 10 having femoral stems 20 of different lengths. According to a preferred version, the stem module 2 has at least two weaknesses 28: in detail, a first weakness 28 is more distal with respect to the end wall 23 and allows a tibial spacer 10 to be obtained having a femoral stem 20 with a greater length; vice versa, a second weakness 28, more proximal with respect to the end wall 23, allows a femoral spacer 20 to be obtained having a femoral stem 20 with a shorter length.

According to the version of the stem module 2 comprising the first and the second stem half-modules 21, 22, the weaknesses 28 are made on at least one between the first and second stem half-modules 21, 22, (Figure 3B) preferably on both; the weaknesses made on each stem half-module 21, 22 can be coupled to allow the facilitated breakage of the stem module 2 in preferential zones.

Weaknesses 28 corresponding to localised thinning can be positioned differently in the stem module 2. For example, the weaknesses 28 may be positioned at the flanges 25, in addition to or alternatively to the weaknesses 28 described above.

Further, the stem module 2 of the assembly 1 according to this invention comprises a coupling site 24 shaped for receiving the closing element 3. In other words, the stem moulding chamber SMC may identify a coupling site 24, configured in use to receive a distal portion 33 of the closing element 3.

More in detail, the coupling site 24 is positioned at the stem moulding chamber SMC and is opposite the end wall 23, considering the longitudinal extension of the stem moulding chamber SMC.

Preferably, the coupling site 24 has a cylindrical shape or, in any case, with a substantially circular transversal cross-section; in particular, the coupling site 24 defines a cylindrical neck 24 configured to receive said closing element 3; the closing element 3, coupled to the stem module 2 and, in particular, inserted inside the coupling site 24, delimits above the stem moulding chamber SMC.

The assembly 1 according to this invention also comprises, in fact, the closing element 3, which can be coupled to the stem module 2 to define and delimit the above-mentioned stem moulding chamber SMC. In particular, the closing element 3, coupled to the stem module 2, delimits above the stem moulding chamber SMC.

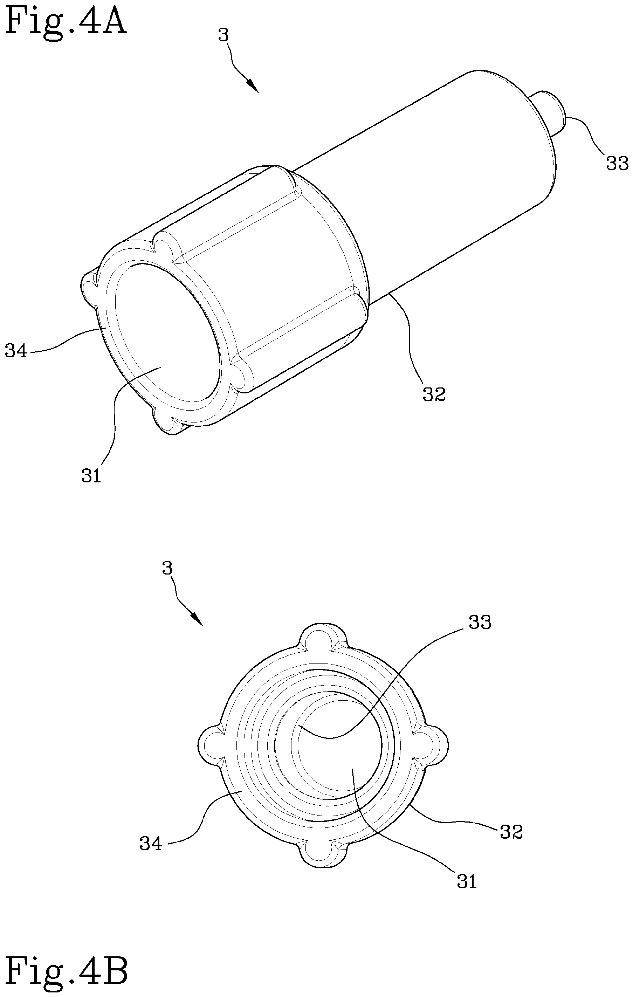

The closing element 3, according to the embodiment illustrated in Figures 4A and 4B, has a tubular extension and comprises a lateral wall 32 delimiting an injection opening 31, for injecting fluid medical cement: the injection opening 31 has a cylindrical shape or, in any case, with a substantially circular transversal cross-section and can be coupled to a spout of a device for dispensing liquid medical cement of the type known to the sector.

Preferably, the lateral wall 32 has a diverging trend between a distal portion 33, in use positioned more proximal to the stem moulding chamber SMC, or, in other words, more inside the cylindrical neck 24 included on the coupling site 24, and a proximal portion 34, in use positioned more distal to the stem moulding chamber SMC, or, in other words, more outside the cylindrical neck 24 included on the coupling site 23. The proximal portion 34 can also be grasped directly or indirectly by the operator for allowing the coupling of the closing element 3 with the stem module 2.

The stem module 2, thanks to the presence of the opening 231 identified at the end wall 23, allows a reinforcing core 4 to be partly positioned inside the stem moulding chamber SMC and to be partly protruding from said stem moulding chamber SMC.

The reinforcing core 4 is, in other words, inserted inside the opening 231 so as to be partly positioned inside the stem moulding chamber SMC, as described in detail below.

The assembly 1 according to this invention comprises, in fact, the reinforcing core 4, which can be partly positioned in the stem moulding chamber SMC and is shaped for passing through the opening 231 of the element of the stem module 2, coming partly out from the stem moulding chamber SMC. In particular, the metal core 4 is positioned inside the stem moulding chamber SMC in such a way that an end portion, which will hereinafter be referred to as the constraining portion 41, protrudes from the stem module 2 passing through the opening 231.

The reinforcing core 4 forms a reinforcing element for the femoral stem 20 of the femoral spacer 10; moreover, the constraining portion 41 allows the femoral stem 20 to be connected to the condylar component 30, interposing a quantity of fluid medical cement between the femoral stem 20 and the condylar component 30, at said constraining portion 41, as described below. Further, said reinforcing core 4 represents a reinforcing element at the interface between femoral stem 20 and condylar component 30 in the femoral spacer 10.

Considering the structure, the reinforcing core 4, preferably made of metal, is elongate in shape, that is to say, it extends mainly along a specific longitudinal direction, between the constraining portion 41, corresponding to a distal end portion, and an insertion portion 42, corresponding to a proximal end portion.

The insertion portion 42 is in use positioned inside the stem moulding chamber SMC, whilst the constraining portion 41 is in use protruding from the stem moulding chamber SMC or, in other words, it is in use protruding from the stem module 2.

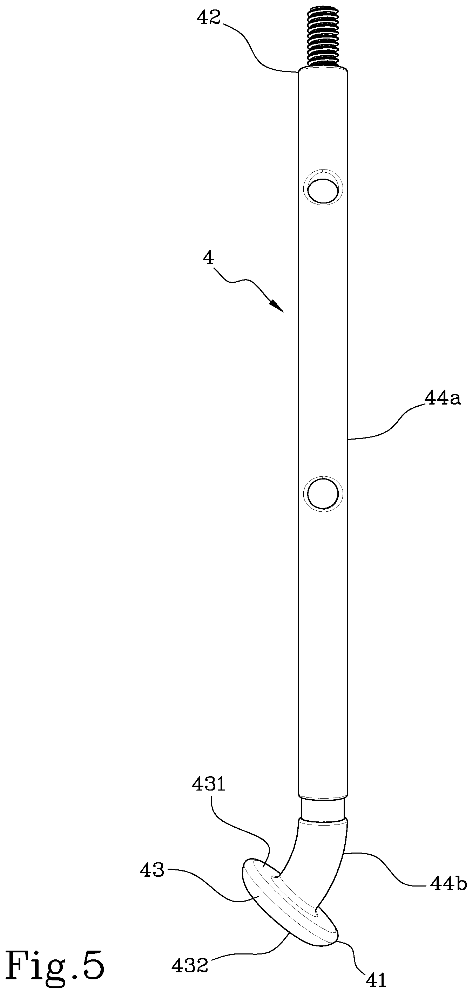

With reference to the embodiment illustrated in Figure 5, the reinforcing core 4 has an elongate portion 44a, rectilinear, and a curved portion 44b, extending at the constraining portion 41; the elongate portion 44a of the reinforcing core 4 has a greater length than the curved portion 44b. In other words, the longitudinal direction of the reinforcing core 4 is identified by the elongate portion 44a. The curved portion 44b, diverting from the longitudinal direction, constitutes a hooked structure, ending with a wide portion 43. In other words, the reinforcing core 4 has a portion with a greater thickness at the constraining portion 41.

The elongate portion 44a is preferably cylindrical in shape.

According to a first embodiment, the wide portion 43 is substantially disc-shaped, positioned transversally relative to the curved portion 44b; according to a preferred embodiment, the wide portion 43 has a first wall 431, which may be substantially flat and positioned transversally to the curved portion 44b, and a second wall 432, adjacent to the first wall 431, which may have a convex shape. For example, the second wall 432 may have the shape of a dome, preferably a flattened dome. Alternatively, the wide portion 43 may be mushroom-shaped.

The constraining portion 41, therefore, thanks to the presence of the wide portion 43 connected to the curved portion 44b defines a hook-shaped connection site, at which it is possible to position a quantity of fluid medical cement: placing a condylar component 30 alongside the femoral stem 20 and, in particular, placing the condylar component 30 alongside said connection site made by the wide portion 43 covered by fluid medical cement, it is possible to cement said condylar component 30 to said femoral stem 20, forming the femoral spacer 10 according to this invention.

Advantageously, thanks to the connection site defined in this way it is possible to make the coupling and combine femoral stems 20 and condylar components 30 having different shapes, without the need for specific coupling structures and specific couplings between said femoral stem 20 and said condylar component 30.

The reinforcing core 4 according to the embodiment of Figure 5 is intended to be located partly inside a rectilinear stem moulding chamber SMC, according to one of the embodiments described above. In this way, it is possible to make the femoral spacer 10 having an elongate and rectilinear reinforced femoral stem 20.

According to an alternative embodiment, not illustrated in the accompanying drawings, the reinforcing core 4 has a proximal stretch at the insertion portion 42, and a distal stretch at the constraining portion 41, having respective axes parallel to the longitudinal direction; more in detail, the proximal stretch and the distal stretch are offset from each other and connected by a connecting stretch. Preferably, the proximal stretch, the distal stretch and the connecting stretch have a substantially constant transversal cross section.

The reinforcing core 4 thus shaped is intended to be partly located inside the stem moulding chamber SMC having the proximal stretch and the distal stretch parallel to the longitudinal direction and offset from each other, said proximal stretch and said distal stretch being connected by an intermediate connecting stretch: in particular, the proximal stretch, the connecting stretch and the distal stretch are inserted respectively in the proximal stretch, in the intermediate stretch and in the distal stretch of the stem moulding chamber SMC.

In this way, it is possible to obtain a femoral spacer 10 having a femoral stem 20 having the proximal portion and the distal portion parallel to the longitudinal axis and offset.

Further, the reinforcing core 4 may comprise an adapter element 4, which can be coupled to the reinforcing core 4 at its proximal end 42; the adapter element is elongate: preferably, the adapter element is rectilinear and substantially cylindrical in shape. The adapter element, through the coupling with the reinforcing core 4, advantageously allows a modular reinforcing core 4 to be obtained, elongate in the longitudinal direction and effectively reinforcing femoral stems 20 having different lengths. In other words, the modular reinforcing core 4 comprises the reinforcing core 4 and the adapter element coupled and aligned parallel to the longitudinal direction.

For example, the proximal end 42 may have a protruding threaded element, which can be coupled to a recessed peripheral portion of the adapter element: screwing the adapter element to the reinforcing core 4 obtains the modular reinforcing core 4.

The reinforcing core 4 may be used for reinforcing femoral stems 20 with smaller lengths; alternatively, the reinforcing core 4, coupled to the adapter element, may be used for reinforcing femoral stems 20 with greater lengths.

In other words, the assembly 1 according to this invention allows an operator to select just the reinforcing core 4 or to couple the adapter to the reinforcing core 4, so as to adequately reinforce a femoral spacer 10 having a femoral stem 20 of a desired length. For this reason, the femoral spacers 10 having femoral stems 20 with greater lengths can also be adequately reinforced, avoiding the more proximal portions from being less resistant and, therefore, more fragile than the distal portions.

The assembly 1 according to this invention also comprises a condylar module, identifying a condylar moulding chamber CMC for making the condylar component 30, designed to be constrained to the reinforcing core 4 at the constraining portion 41 and, preferably, at the wide portion 43. The condylar moulding chamber CMC is a space inside the condylar module and is formed by the shape of a relative inner surface. The condylar moulding chamber CMC is designed to receive a quantity of medical cement in a fluid form and to allow the solidification to obtain the condylar component 30, which, in use, is configured to restore the functionality of the distal part of the femur and, in particular, of the distal femoral epiphysis.

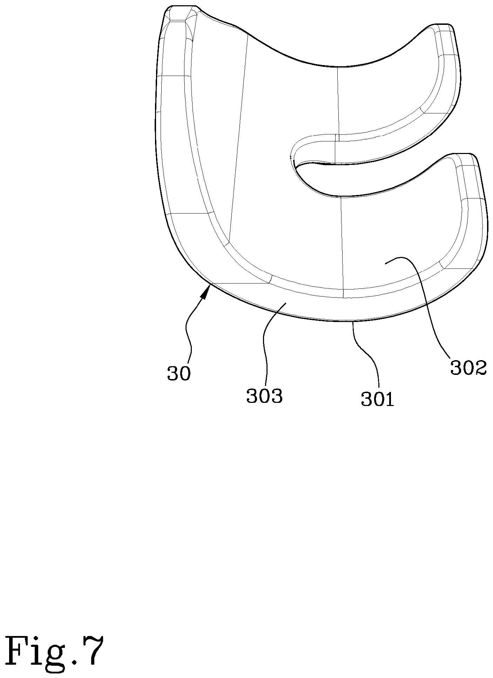

The condylar moulding chamber CMC is therefore internally shaped to reproduce during moulding the anatomy of a condylar component 30 (Figure 7); preferably, the condylar component 30 made inside the condylar moulding chamber CMC comprises two tuberosities alongside each other and corresponding, respectively, to a medial femoral condyle and a lateral femoral condyle of the distal femoral epiphysis; in other words, the condylar component 30 made using the condylar module corresponds to the distal portion of femur which has a corresponding proximal tibial epiphysis, to make a knee joint.

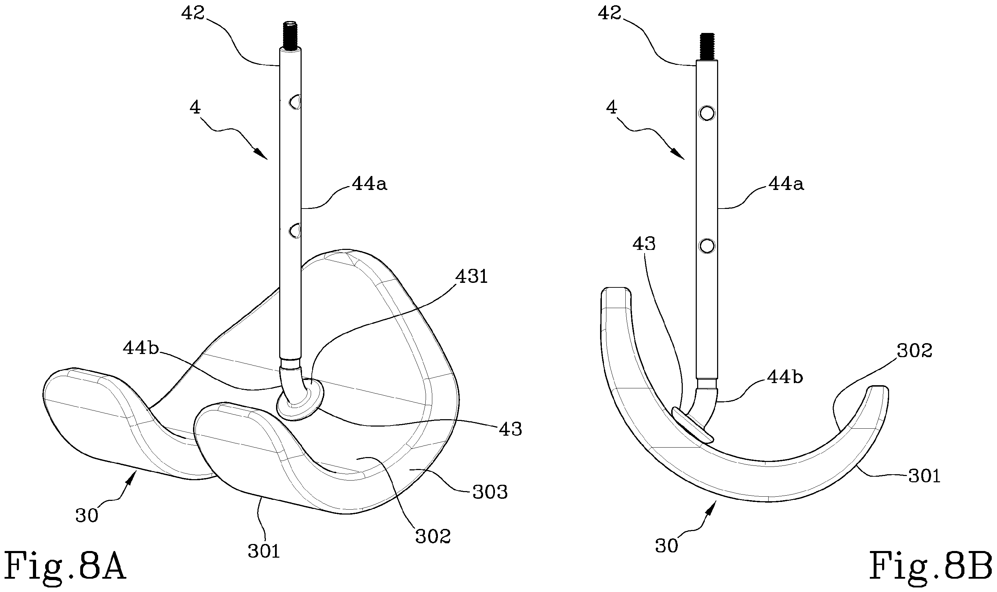

Once the solidification of the medical cement has been performed inside the condylar moulding chamber CMC, the condylar component 30 obtained can be extracted from the condylar module and can then be constrained to the femoral stem 20 at the constraining portion 41 of the reinforcing core 4. In particular, the condylar component 30 is constrained at the wide portion 43 (Figures 8A and 8B), interposing medical cement in a fluid form which, solidifying, cements the condylar component 30 to the femoral stem 20.

The assembly 1 according to this invention also comprises centring means 26, 6 for positioning the reinforcing core 4 in a predetermined position relative to the stem moulding chamber SMC.

In detail, the centring means may comprise one or more seats 26 for centring the reinforcing core 4 made at the inner surface of the stem moulding chamber SMC. More in detail, in order to keep the reinforcing core 4 correctly positioned inside the stem moulding chamber SMC and to prevent it from moving in an unpredictable and undesired manner during the injection of the fluid medical cement, the assembly 1 comprises, at the stem moulding chamber SMC, one or more centring seats 26 made from blind holes transversely oriented relative to the longitudinal direction and connected to the stem moulding chamber SMC. According to the embodiment of the stem module 2 comprising the first and second stem half-modules 21, 22, the centring seats 26 are preferably positioned in a specular manner, so as to be facing each other and aligned when the first and second stem half-modules 21, 22 are coupled to define the stem moulding chamber SMC.

The centring means may comprise one or more centring pins 6 of elongate shape, designed to be inserted in the centring seats 26, prepared, in use, for maintaining the reinforcing core 4 in the correct positioning. The centring pins 6 have an elongate shape, between a constraining end and a free end: the constraining end can be coupled to the reinforcing core 4 in such a way that said centring pin 6 is substantially perpendicular to the longitudinal direction of the reinforcing core 4, whilst the free end is shaped for inserting inside the blind holes making the centring seats 26. As many centring pins 6 may be coupled to the reinforcing core 4 perpendicularly to the longitudinal direction as there are centring seats 26 in communication with the stem moulding chamber SMC. In other words, the centring pins 6 of the assembly 1 according to this invention operate as means for anchoring the reinforcing core 4 to the stem module 2 and, in particular, to an inner wall of said stem module 2 at the stem moulding chamber SMC, in such a way that the reinforcing core 4 maintains the correct positioning and does not undergo undesired movements during injection of the fluid cement, for example through the injection opening 31, and before its complete solidification.

Lastly, the assembly 1 according to this invention comprises a plurality of fixing means 7. In order to keep the stem half-modules 21, 22 next to each other during the injection of the cement and to prevent unwanted relative movements between them, the holes 27 made at the flanges 25 may be advantageously engaged by the fixing means 7 included in the assembly 1 according to this invention.

The fixing means 7, in particular, operate between a constraining configuration, wherein they determine the coupling, respectively, of the first to the second stem half-modules 21, 22 and a release configuration, wherein they determine the uncoupling of the first from the second stem half-modules 21, 22. Preferably, the fixing means 7 have a central reduction in area designed to be broken in the passage from the constraining configuration to the release configuration. Fixing means 7 operating as indicated are known to an expert in the trade.

Another object of the invention is a kit for making in an operating room a plurality of intramedullary spacers 20 with different dimensions and configurations: in effect, intramedullary spacers 20 identical in the shape and in the dimensions may be well performing for some patients and, on the other hand, they may not be equally satisfactory for other patients due to factors such as differences in height, age, state of health of the bone, level of physical activity between these patients.

In order to overcome this limitation, the kit according to this invention comprises a plurality of stem modules 2, identifying stem moulding chambers SMC having different dimensions and/or three-dimensional shapes.

The stem modules 2 may vary, for example, in terms of diameter and length of the stem moulding chamber SMC, to make intramedullary spacers 20 of different thicknesses or lengths.

Alternatively or in addition, the stem modules 2 may vary in terms of three-dimensional configuration of the stem moulding chamber SMC: in this second case, the operator can choose, for example, between a stem module 2 making an intramedullary spacer 20 which is substantially rectilinear or cylindrical and an intramedullary spacer 20 with offset, optionally also with different offsets, on the basis of data collected in the pre-operation phase relative to the anatomy of the patient.

In detail, each stem module 2 inside the kit according to this invention comprises an end wall 23 delimiting below the stem moulding chamber SMC; according to an alternative embodiment of the kit according to this invention, the stem module 2 has an opening 231 for connecting said stem moulding chamber SMC with an outside environment.

Optionally, the kit according to this invention comprises a closing element 3 which can be coupled to each stem module 2 to delimit above the stem moulding chamber SMC: in particular, a same closing element 3 may be coupled to a site 24 for coupling stem modules 2 defining stem moulding chambers SMC of different dimensions and/or three-dimensional shapes. Further, the kit according to this invention comprises one or more reinforcing cores 4 of different dimensions and/or three-dimensional shapes, said reinforcing cores 4 being, in use, located inside or partly inside the stem moulding chamber SMC.

For example, the kit may comprise reinforcing cores 4 with different lengths and/or thicknesses and/or offsets.

The kit comprising a plurality of stem modules 2 and a plurality of cores which can be located inside the stem moulding chamber SMC can be used for moulding and making temporary intramedullary nails 20 with different dimensions and three-dimensional shapes.

Alternatively, the kit comprising the stem modules 2 and the reinforcing cores 4 which can be positioned partly inside the stem moulding chamber SMC and partly protruding from said stem moulding chamber SMC allow the moulding of femoral stems 20, or positioning and fixing stems which can be used for making femoral spacers 10 of different dimensions or three-dimensional configurations.

According to an embodiment, the kit according to this invention further comprises one or more condylar modules identifying condylar moulding chambers CMC with different dimensions and/or shapes; for example, the kit may comprise condylar modules for making condylar components having different dimensions and/or thickness and/or three-dimensional shape: in this case, the operator can select, for example, a condylar module making a condylar component 30 of specific dimensions and thickness, on the basis of data collected in the pre-operating phase relative to the anatomy of the patient.

Advantageously, the kit according to the invention allows the selection between a plurality of temporary intramedullary nails 20, femoral stems 20 or femoral spacers 10 which can be made, being particularly versatile and effective in responding to specific anatomical requirements. Advantageously, taking into account said specific anatomical requirements, the kit according to this invention allows the subsequent implant step to be facilitated, avoiding or, in any case, limiting undesired events, including the breakage of the temporary intermedullary nail 20 or of the femoral stem 20 due to impact with portions of cortical bone surrounding the femoral canal, or breakage of the cortical bone following the insertion of a femoral spacer 10 with incorrect dimensions.

This invention also relates to a method for making a temporary intramedullary spacer 20 comprising a reinforcing core 4.

The method proposed can be implemented using the assembly 1 described above.

The method according to this invention comprises a plurality of steps set out below in the preferred order. However, a different order of execution of the steps described below is not excluded.

The embodiment method according to this invention comprises, firstly, the step of providing a stem moulding chamber SMC for making the intramedullary spacer 20; in particular, said stem moulding chamber SMC may be identified by coupling the above-described first and second stem half-modules 21, 22 to form the stem module 2.

Considering the kit described above, the method according to the invention may comprise the preliminary step of selecting a stem module 2 between the plurality of stem modules 2.

The method therefore comprises a step of preparing a reinforcing core 4 and, subsequently, positioning the reinforcing core 4 at least partly inside the stem moulding chamber SMC.

Considering the kit described above, the method according to this invention may comprise the step of selecting a reinforcing core 4 from a plurality of reinforcing cores 4.

In detail, the reinforcing core 4 is positioned inside the stem moulding chamber SMC, to make a temporary intramedullary nail 20 which can be inserted in a medullary canal.

Alternatively, the reinforcing core 4 is positioned partly inside the stem moulding chamber SMC, keeping the constraining portion 41 protruding from the stem module 2 and positioning the insertion portion 42 inside the stem moulding chamber SMC, to make a femoral stem 20. In other words, after having coupled first and second stem half-modules 21, 22, the reinforcing core 4 has the insertion end 42 inside the stem moulding chamber SMC and the constraining end 41 protruding from said stem moulding chamber SMC and coming out from it through the opening 231. In both the cases introduced, before coupling the first stem half-module 21 to the second stem half-module 22, it is necessary to prepare one or more centring pins 4, coupling each centring pin 6 to the reinforcing core 4, keeping said centring pin 6 substantially perpendicular to the longitudinal direction, and, therefore, positioning each centring pin 6 coupled to the reinforcing core 4 inside a respective blind hole making a centring seat 26. The method according to this invention also comprises the step of coupling a closing element 3 to the stem module 2, to define above the stem moulding chamber SMC; for example, the closing element 3 may be positioned at a coupling site 24 identified at the stem module 2.

The method according to this invention comprises the step of preparing a quantity of fluid medical cement and the subsequent step of injecting said quantity of fluid medical cement inside the stem moulding chamber SMC, to fill said stem moulding chamber SMC.

The injection of the medical cement may occur through a dispensing device of the type known to the sector and having a spout which can be coupled to an injection opening 31 of the closing element 3.

After filling the stem moulding chamber SMC with fluid medical cement, the dispensing device is removed from the injection opening 31.

The method according to this invention also comprises the step of waiting for a predetermined time for the solidification of the medical cement inside the stem moulding chamber SMC, making the femoral stem 20 comprising the protruding reinforcing core 4.

The method according to this invention comprises the step of releasing the temporary intramedullary nail 20 or the femoral stem 20 made of solidified medical cement; in particular, in order to release the temporary intramedullary nail 20 or the femoral stem 20 it is necessary to uncouple the first from the second stem half-module 21, 22. If there are fixing means 7, before uncoupling the first and second stem half-modules 21, 22 it is necessary to move said fixing means 7 from the coupling configuration to the release configuration, for example, by breaking them at a central ridge. Optionally, in order to obtain a femoral spacer 10 comprising a femoral stem 20 having a reinforcing core 4 protruding from said femoral stem 20 and constrained to a condylar component 30 through a quantity of bone cement, the method according to this invention comprises a step of making available a condylar component 30. According to an embodiment, the condylar component 30 is made by solidification of fluid medical cement injected inside a condylar moulding chamber CMC identified by a condylar module.

Considering the kit described above, the method according to this invention may comprise the step of selecting a condylar module from a plurality of condylar modules.

Lastly, the method according to this invention comprises the step of coupling the condylar component 30 to the femoral stem 20 interposing a further quantity of fluid medical cement, making the femoral spacer 10.

In particular, the coupling between the femoral stem 20 and the condylar component 30, as described above, is made by applying a quantity of fluid medical cement at the protruding portion 41; alternatively or in addition, the further fluid medical cement can be positioned at a proximal surface of the condylar component 30. The condylar component 30 is kept alongside the protruding portion 41 of the femoral stem 20 until solidification of the further medical cement interposed between femoral stem 20 and the condylar component 30, to make the femoral spacer 10.

The method according to this invention allows, firstly, an intramedullary spacer 20 made of medical cement to be obtained designed to be inserted in a medullary canal to make a femoral nail 20 in use configured to align bone segments separated by a broken fracture.

The method according to this invention also allows a femoral spacer 10 to be obtained in a fast and effective manner for a temporary knee prosthesis made of medical cement: advantageously, the method according to this invention allows the combination in a fast, effective and versatile manner of femoral stems 20 and condylar components 30 having different shapes, to make a wide variety of femoral spacers 10 which are able to satisfy the specific anatomical needs of a patient.

Inside the femoral spacer 10 according to the invention, the condylar component 30 is in use designed to restore the functionality of the distal femoral epiphysis, whilst the femoral stem 20 is in use designed to be inserted inside the femoral canal of the patient, acting as a constraining and stabilising element for the femoral spacer 10.

The condylar component 30 has a distal surface 301 opposite a proximal surface 302 and connected to said proximal surface 302 through a lateral wall 303 defining the thickness of the condylar component 30: the distal surface 301 is shaped for articulating with a corresponding tibial spacer to obtain a temporary knee prosthesis.

The femoral stem 20 protrudes from the condylar component 30 at the proximal surface 302, that is to say, opposite the distal surface 301.

The femoral spacer 10 according to this invention has a femoral stem 20 having a proximal portion constrained to the proximal surface 302 of the condylar component 30, and a distal portion away from the proximal surface 302.

As already explained above, according to a first embodiment, the proximal portion and the distal portion are aligned along the longitudinal direction; therefore, the femoral stem 20 has an elongate shape along said longitudinal direction.

Alternatively, the proximal portion and the distal portion may be parallel to the longitudinal direction and offset from each other: according to this embodiment, the proximal portion and the distal portion are connected by an intermediate connecting portion, to make a femoral stem 20 with offset.

## Claims

1. An assembly (1) for making an intramedullary spacer (20) made of medical cement, designed to be inserted in a medullary canal, comprising a stem module (2) and a reinforcing core (4),
wherein the stem module (2) comprises an end wall (23) delimiting below a stem moulding chamber (SMC); and
wherein the reinforcing core (4) can be at least partly positioned in the stem moulding chamber (SMC).

2. The assembly according to the preceding claim, comprising a closing element (3) which can be coupled to the stem module (2) to delimit above the stem moulding chamber (SMC).

3. The assembly according to any one of the preceding claims, wherein the end wall (23) has an opening (231), for putting in communication the stem moulding chamber (SMC) with an outside environment;

4. The assembly according to the preceding claim, wherein the reinforcing core (4) has a constraining portion (41) protruding from the stem moulding chamber (SMC) through the opening (231) of the end wall (23), to make a femoral stem (20) designed to be inserted in a femoral medullary canal.

5. The assembly according to any one of the preceding claims, comprising a condylar module made of medical cement defining a condylar moulding chamber (CMC) for making a condylar component (30) which can be coupled with the femoral stem (20) for making a femoral spacer (10) of a temporary knee prosthesis, said condylar moulding chamber (CMC) being designed to be constrained to the reinforcing core (4) at the constraining portion (41).

6. The assembly according to any one of claims 1 to 5, wherein the stem moulding chamber (SMC) has a rectilinear shape.

7. The assembly according to any one of claims 1 to 5, wherein the stem moulding chamber (SMC) has a first stretch and a second stretch offset from each other.

8. The assembly according to any one of claims 4 to 7, wherein the stem module (2) has a coupling site (24) comprising a cylindrical neck (24) configured for receiving a distal portion (33) of the closing element (3).

9. The assembly according to any one of the preceding claims, wherein the stem module (2) comprises a first stem half-module (21) and a second stem half-module (22), which can be coupled to the first stem half-module (21) to define the moulding chamber (SMC).

10. The assembly according to any one of the preceding claims, wherein the closing element (3) has a tubular extension and comprises a side wall (32) delimiting an injection opening (31) which in use can be coupled to a spout for dispensing fluid medical cement.

11. The assembly according to the preceding claim, wherein the side wall (32) has a diverging shape between a distal portion (33) in use positioned more proximal with respect to the stem moulding chamber (SMC), and a proximal portion (34), in use positioned more distal with respect to the stem moulding chamber (SMC).

12. The assembly according to any one of the preceding claims, wherein the reinforcing core (4) has an elongate extension between a constraining portion (41) and an insertion portion (42), said insertion portion (42) being in use positioned inside the stem moulding chamber (SMC).

13. The assembly according to the preceding claim, wherein the reinforcing core (4) is rectilinear.

14. The assembly according to claim 12, wherein the reinforcing core (4) has an offset first stretch and second stretch.

15. The assembly according to any one of claims 12 to 14, wherein the reinforcing core (4) has a wide portion (43) at the constraining portion (41).

16. The assembly according to claim 1, comprising centring means (26, 6) for positioning the reinforcing core (4) in a predetermined position with respect to the stem moulding chamber (SMC).

17. The assembly according to the preceding claim, wherein the centring means comprise one or more centring seats (26) made from blind holes made in the stem moulding chamber (SMC).

18. The assembly according to claim 16 or 17, wherein the centring means comprise centring pins (6) which can be coupled to the reinforcing core (4) and designed to be inserted in respective centring seats (26).

19. A kit for making a plurality of intramedullary spacers (20) in an operating room, said kit comprising:
a plurality of stem modules (2) of an assembly (1) according to claim 1 or 2, of different dimensions and/or shapes; and
one or more reinforcing cores (4) of an assembly (1) according to claim 1, of different dimensions and/or shapes.

20. The kit according to the preceding claim, comprising an element (3) for closing an assembly (1) according to claim 1.

21. The kit according to claim 19 or 20, comprising one or more condylar modules (5) according to claim 5 defining condylar moulding chambers (CMC) of different dimensions and/or shapes.

22. A method for making an intramedullary spacer (20) made of medical cement, designed for being inserted in a medullary canal, said method comprising the steps of:
- Providing a stem moulding chamber (SMC) for making the intramedullary spacer (20);
- Preparing a reinforcing core (4);
- Positioning the reinforcing core (4) at least partly inside the stem moulding chamber (SMC),
- Preparing a quantity of medical cement in liquid form;
- Injecting the medical cement in a fluid form to fill the stem moulding chamber (SMC);
- Waiting a predetermined time for the solidification of the medical cement inside the stem moulding chamber (SMC), making the intramedullary spacer (20).

23. The method according to claim 22, wherein the reinforcing core (4) is partly positioned inside the stem moulding chamber (SMC) keeping a constraining portion (41) protruding, to make a femoral stem (20) designed to be inserted in a femoral medullary canal.

24. The method according to claim 23, comprising the steps of:
- Providing a condylar component (30);
- Coupling the condylar component (30) to the femoral stem (20) at the constraining portion (41) interposing a further quantity of medical cement;
- Waiting a predetermined time for the solidification of the further medical cement, making a femoral spacer (10) comprising the femoral stem (20) and the condylar component (30).

25. An intramedullary spacer (20) made of medical cement having a reinforcing core (4) coated with medical cement designed to be inserted in a medullary canal to form a femoral nail (20) configured to align bone segments separated by a broken down fracture.

26. A femoral spacer (10) for a temporary knee prosthesis made of medical cement comprising a femoral stem (20) and a condylar component (30), wherein the femoral stem (20) has a reinforcing core (4) protruding and constrained to the condylar component (30) by interposition of a quantity of bone cement.

27. The femoral spacer (10) according to the preceding claim, wherein the femoral stem (20) has a proximal portion and a distal portion which are aligned with each other.

28. The femoral spacer (10) according to claim 26, wherein the femoral stem (20) has a proximal portion and a distal portion offset from each other.
